# EUROPEAN PATENT SPECIFICATION

(11) **EP 3 698 840 B1**
(45) Date of publication and mention of the grant of the patent: **26.05.2021**
(21) Application number: 20157608.9
(22) Date of filing: 17.02.2020
(51) Int. Cl.: A61M 25/00, A61B 18/14

(54) **TRANSESOPHAGEAL CATHETER WITH CARBON DIOXIDE DELIVERY SYSTEM FOR THERMAL PROTECTION OF ESOPHAGUS**
TRANSÖSOPHAGEALER KATHETER MIT KOHLENDIOXIDABGABESYSTEM ZUM THERMISCHEN SCHUTZ DER SPEISERÖHRE
CATHÉTER TRANS SOPHAGIEN DOTÉ D'UN SYSTÈME D'ADMINISTRATION DE DIOXYDE DE CARBONE POUR LA PROTECTION THERMIQUE DE L' SOPHAGE

(30) Priority: 15.02.2019 US 201916276685
(43) Date of publication of application: 26.08.2020
(73) Proprietor: BIOSENSE WEBSTER (ISRAEL) LTD., 2066717 Yokneam (IL); Ethicon, Inc., Somerville, NJ 08876 (US)
(72) Inventor: COHN, William, Houston, TX Texas 77021 (US); HIGHSMITH, Debby Esther, Irwindale, CA California 92677 (US); KUHN, Matthew, Houston, TX Texas 77021 (US); WONG, Fergus, Houston, TX Texas 77021 (US)
(74) Representative: Small, Gary James

(56) References cited:
- WO-A1-2015/148541
- US-A1- 2009 069 875
- US-A1- 2015 045 825
- US-A1- 2017 333 122
- US-A1- 2017 360 503

## Description

### CROSS-REFERENCE TO RELATED APPLICATIONS

This patent application is a continuation-in-part application of United States Patent Application Number 16/127,461 filed September 11, 2018 which claims the benefit of United States Provisional Application Number 62/631359 filed February 15, 2018.

### BACKGROUND OF THE INVENTION

### 1. Field of the Invention

The present invention relates to systems for preventing esophageal damage and fistula formation, and more particularly to systems for preventing esophageal damage and fistula formation after intra-cardiac catheter ablation of the left atrium.

### 2. Discussion of the Related Art

Cardiac arrhythmias, and atrial fibrillation, persist as common and dangerous medical ailments, especially in the aging population. In patients with normal sinus rhythm, the heart, which is comprised of atrial, ventricular, and excitatory conduction tissue, is electrically excited to beat in a synchronous, patterned fashion. In patients with cardiac arrhythmias, abnormal regions of cardiac tissue do not follow the synchronous beating cycle associated with normally conductive tissue as in patients with normal sinus rhythm. Instead, the abnormal regions of cardiac tissue aberrantly conduct to adjacent tissue, thereby disrupting the cardiac cycle into an asynchronous cardiac rhythm. Such abnormal conduction has been previously known to occur at various regions of the heart, for example, in the region of the sino-atrial (SA) node, along the conduction pathways of the atrioventricular (AV) node and the Bundle of His, or in the cardiac muscle tissue forming the walls of the ventricular and atrial cardiac chambers.

Atrial fibrillation affects millions of Americans. Patients with atrial fibrillation have a significantly increased risk of suffering stroke, heart attack, leg loss, and other adverse events. Intra-cardiac catheter ablation has emerged as the dominant therapy for treating atrial fibrillation. By creating full-thickness lines of scar tissue in the left atrium, the chaotic waves of electrical activity necessary to maintain atrial fibrillation are isolated, and the patient's heart rhythm converts to a regular organized one. The lines of scar tissue must be full-thickness, which is to say, must extend from the inner lining of the heart, the endocardium, all the way through the entire thickness of the atrial wall to the outer lining, the epicardium. If the scar tissue is only partial-thickness, the electrical waves can still propagate around the scar.

Biosense Webster is a global leader in the field of treating atrial fibrillation. The Biosense Webster CARTO® 3 system allows accurate mapping of the atrium, navigation inside the atrium with an ablation catheter, and creation of full-thickness lesions. Despite the sophistication of the Biosense Webster system, avoiding esophageal damage and occasional post procedural development of an atrial-esophageal fistula remains a challenge. This complication occurs because of the proximity between the esophagus, the swallowing tube that connects the mouth or more accurately, the pharynx to the stomach, and the back wall of the left atrium.

When creating the pattern of left atrial scar that has been identified as most effective in converting atrial fibrillation, it is necessary to create a line that runs transversely across the back wall of the left atrium. During creation of this line, the esophagus may be scarred. This is particularly challenging because usually there is no evidence during the procedure that suggests the esophagus has been injured. The classic presentation is that of a patient who returns two weeks after a "successful" ablation with a low-grade fever of unknown origin or a small stroke. On further investigation, it is revealed that the patient has developed endocarditis, an infection of the heart and heart lining, resulting from drainage of esophageal contents into the heart, or that the patient has had a stroke which resulted from a small bubble of air arising from the esophageal lumen that has passed into the left atrium. Regardless of presentation, the development of an atrial esophageal fistula or abnormal passageway is a potentially serious complication. Patients generally must undergo a major thoracic operation if crisis is to be averted.

Catheter ablation for converting atrial fibrillation to normal organized rhythm requires the successful creation of full-thickness lines of scar tissue in a prescribed pattern throughout the left atrium. One of the lines, by necessity, crosses the back wall where the left atrium and the esophagus are in close proximity. In a significant percentage of cases, the esophagus is inadvertently injured during creation of this burn, which on occasion (0.5 percent to 1.5 percent) results in the delayed formation (approximately two weeks later) of a left atrial-to-esophageal fistula. If the burns do not involve the full thickness of the left atrium wall, the therapy is unlikely to be successful. Electric current may still travel through the partial thickness of living heart muscle and the atrial fibrillation persists. Because of increased awareness of this complication, electrophysiologists less aggressively ablate tissue as they cross the back wall, and fewer patients benefit from successful conversion to regular rhythm as a result. There is consensus among electrophysiologists that a solution is needed to allow aggressive treatment of the left atrium without risk of this potential complication.

Others have proposed solutions. The two main types are: 1) devices that utilize a shaped balloon, rod, or nitinol structure in an effort to pull the esophagus away from the back wall of the left atrium so the electrophysiologist can be more aggressive creating posterior burns; or 2) devices passed down the esophagus that measure temperature, impedance, or other metrics to inform the electrophysiologist when it is safe to burn and when it is not, or when the esophagus is heating up during ablation so the electrophysiologist can stop immediately.

The challenges with the first type include the need for the electrophysiologist to manipulate the esophagus, something with which they typically have little familiarity, and the challenges with moving the esophagus. The two structures, the esophagus and the left atrium, are immediately adjacent to each other in an air-tight space. As one pulls the esophagus away from the left atrium, the atrium is pulled somewhat in conjunction with the esophagus. Moreover, there have been reports of esophageal injury while trying to pull the esophagus by applying traction to it from within its lumen. These injuries include occasional esophageal hematomas, which may require surgical treatment.

The challenges with esophageal temperature monitoring center around its reactive nature. This monitoring only allows the electrophysiologist to determine that the esophagus lumen has increased in temperature, indicating that a thermal insult to the esophageal wall has already occurred. Although this measurement allows the electrophysiologist to immediately stop burning and in so doing, limit the extent of the thermal exposure, the measurement does nothing to prevent such injury from happening.

Accordingly, there exists a need for a reliable system for preventing esophageal fistula formation during intra-cardiac catheter ablation of the left atrium.

### SUMMARY OF THE INVENTION

The present invention is described in the claims and is directed to system(s) wherein sufficient volumes of carbon dioxide gas is injected into the layer of connective tissue that sits between the esophagus and the back wall of the left atrium to create a protective layer of insulation that will prevent thermal injury to the esophagus while intentionally creating full-thickness burns in the left atrium. The present invention overcomes a number of the limitations associated with the prior art as briefly described above.

In accordance with one aspect, the present invention is directed to a catheter-based system for the delivery of carbon dioxide to the fibro-fatty tissue between the esophagus and the heart for the prevention of esophageal damage and/or fistula during intra-cardiac ablation of the left atrium. The system comprising a transesophageal catheter configured for introduction through the esophagus of a patient, the transesophageal catheter including a needle delivery device and associated needle and an anchoring mechanism; a gas supply configured to deliver gas to the needle through the transesophageal catheter, the gas supply being connected at the proximal end of the transesophageal catheter; and a feedback control system configured to regulate the pressure and flow rate of the gas from the gas supply to the needle.

Carbon dioxide insufflation, unlike these other approaches, creates an insulating sleeve around the esophagus, in effect isolating the esophagus from the posterior left atrium wall. The reference "Anatomic Relations Between the Esophagus and Left Atrium and Relevance for Ablation of Atrial Fibrillation," Circulation 2005; 112:1400-1405, describes the heterogeneity with respect to the amount and thickness of fibro-fatty tissue interposed between the esophagus and the left atrium. In almost half of the cadavers they dissected, the thickness is less than 5 mm. When carbon dioxide is injected into this fibro-fatty layer, the tissue inflates, and becomes "emphysematous," a term that describes solid tissue infused with gas. The best analogous example from the non-medical world is from foods, such as cotton candy or marshmallows. Each is made from a small volume of sugar that is increased in volume by infusing room or ambient air. Sugar has a density of 1.6 g/cm³ and marshmallow has a density of about 0.4 g/cm³. The volume is increased by a factor of four (4) by infiltrating with air. Similarly, cotton candy has a density of 0.005 g/cm³. It is over ninety-nine (99) percent air. It is also no coincidence that cotton candy looks like the insulation that home builders use when building energy efficient homes. Trapped gas, that is, gas that is not free to blow away with a slight breeze or movement, is an excellent insulator. That explains why Styrofoam® insulates so well (poly-styrene infused with gas), and why fur coats and down feather jackets are so warm. Trapped gas acts as a superb insulator.

The 3 mm to 6 mm layer of fibro-fatty tissue that separates the posterior left atrium wall from the esophagus will be converted into a thicker layer of gas infused tissue that will surround the esophagus and provide adequate thermal insulation, thereby preventing it from being injured. Carbon dioxide is utilized instead of air to leverage carbon dioxide's water solubility. This has no effect on the ability of carbon dioxide to serve as an insulator as it will behave just like air in this regard, but if carbon dioxide is injected directly into the left atrium, there will be no adverse sequelae. Carbon dioxide is so soluble that it goes readily into solution when pressurized. It makes it highly unlikely to create a carbon dioxide gas embolus, and thereby makes it safe to use inside the left atrium at dosages less than 3 mL/kg. It is important to note that dosages of carbon dioxide less than 3 mL/kg that has been introduced into the cranial circulatory system is tolerated with no neurotoxicity, but the potential to cause embolic stroke in the cranial system does exist (https://www.ncbi.nlm.nih.gov/pmc/articles/PMC4603680/). Because a gas is being injected, the needle to be utilized may be small enough, e.g. on the order of a 27-gauge needle, so that the risk of potential injury to the left atrium or esophagus is essentially non-existent.

### BRIEF DESCRIPTION OF THE DRAWINGS

The foregoing and other features and advantages of the invention will be apparent from the following, more particular description of preferred embodiments of the invention, as illustrated in the accompanying drawings.
Figure 1 is a block diagram representation of an exemplary system for the prevention of esophageal damage and/or fistula during intra-cardiac ablation.
Figure 2 is a diagrammatic representation of the proximal portion of an exemplary catheter system.
Figure 3 is a diagrammatic representation of an exemplary Tuohy Borst valve portion of an injection catheter.
Figures 4A - 4D are diagrammatic representations of the distal portion of a first exemplary catheter system.
Figure 5 is a diagrammatic representation of the distal portion of a second exemplary catheter system.
Figure 6 is a graphical representation of flow rate versus needle penetration depth for various regions of the anatomy.
Figure 7 is a graphical representation of voltage versus needle penetration depth for various regions of the anatomy.
Figure 8A is a diagrammatic representation of a first exemplary transesophageal catheter with an uninflated balloon and undeployed needle.
Figure 8B is a diagrammatic representation of the first exemplary transesophageal catheter with an inflated balloon and deployed needle.
Figure 9A is a diagrammatic representation of a second exemplary transesophageal catheter with an uninflated balloon and deployed needle.
Figure 9B is a diagrammatic representation of a detailed sectional view of a needle biasing component of the second exemplary transesophageal catheter.
Figure 9C is a diagrammatic representation of a first view of the needle of the transesophageal catheter.
Figure 9D is a diagrammatic representation of a second view of the needle of the transesophageal catheter.
Figure 10 is a diagrammatic representation of an indicator wheel.
Figure 11 is a flow diagram of the insufflation process.
Figure 12 is a diagrammatic representation of the slider mechanism of the transesophageal catheter.

### DETAILED DESCRIPTION OF THE PREFERRED EMBODIMENTS

The present invention is directed to system(s) for preventing or minimizing the formation of an esophageal fistula or esophageal tissue damage due to unintended thermal dispersion during intra-cardiac ablation of the left atrium. In the present invention, carbon dioxide is injected or infused into the fibro-fatty tissue that separates the posterior left atrium wall from the esophagus to expand the tissue and create an insulation layer therebetween. With the carbon dioxide infused tissue insulation layer in place, catheter ablation may be utilized to create full-thickness scar tissue with minimal risk of damaging the esophagus and forming an esophageal fistula. A description of experiments given below demonstrate the feasibility and efficacy of the inventive concept.

An eight-animal study was conducted to demonstrate that carbon dioxide could be safely injected through a catheter inserted up the femoral vein to the right atrium and through the right atrial wall into the pericardium to facilitate obtaining pericardial access. The study demonstrated that carbon dioxide may be safely injected into biological tissue. The study also demonstrated that carbon dioxide offers a number of advantages over air, including high solubility, low viscosity, radio-translucency and excellent thermal and electrical insulation qualities. More specifically, carbon dioxide which is fifty-four times more soluble than nitrogen and twenty-eight times more soluble than oxygen, is typically reabsorbed in less than two hours and is highly unlikely to result in gas embolus, even in large quantities, due to its solubility in water. Carbon dioxide has a low viscosity, allowing it to pass through a needle as small as a 33-gauge needle. The puncture from this size needle seals almost immediately after removal, even in the presence of systemic heparin, thereby reducing the likelihood of complications. Carbon dioxide is also visible under X-ray fluoroscopy, thereby allowing for visible confirmation of successful insufflation by creating an outline of the esophagus under X-ray fluoroscopy. Finally, carbon dioxide is a good electrical and thermal insulator which is exactly what is required to protect the esophagus during intra-cardiac catheter ablation.

The eight-animal study was followed with two separate acute animal experiments. In each, the esophagus of a pig was exposed through a left thoracotomy. Because the esophagus does not run behind the left atrium in pigs, it was possible to directly observe the juxta-esophageal tissue as an indicator of the feasibility of carbon dioxide injection to create a protective barrier layer. A carbon dioxide source was connected to a stopcock which allowed a 60-cc syringe connected to a 27-gauge needle to be filled with pure carbon dioxide. The carbon dioxide was injected into the soft tissue surrounding the esophagus. The carbon dioxide immediately dissected through the soft tissue surrounding the esophagus and increased the thickness of the fibro-fatty layer by creating an emphysema (carbon dioxide infused tissue). The carbon dioxide infused through the tissue all the way around the circumference of the esophagus and tracked toward the head and tail as far as the esophagus was exposed. The thickness of the barrier layer was demonstrated by cutting therethrough. The thickness of the gas-infused tissue was visible on X-ray, presenting as a lucent halo around the esophagus. One may also appreciate that the esophagus moved away from the spine due to the circumferential nature of the carbon dioxide emphysema. Essentially, the carbon dioxide emphysema isolates the esophagus from all other anatomical structures.

Upon completion of the pig studies, two human cadaver studies were conducted to demonstrate the feasibility of forming an insulation layer around the esophagus by creating an emphysema. In both cadavers, a simple investigation was conducted by injecting 120 cc (two complete 60 cc syringes) of carbon dioxide through the back wall of the left atrium. This was also done under direct vision, as the heart in each of the cadavers had been dissected. This study was an endeavor to demonstrate the feasibility of the concept of forming an insulation layer by creating an emphysema or separation. After cutting through the posterior left atrium wall, it was observed that emphysematous tissue between the left atrium and the esophagus formed as it did in the animal studies utilizing carbon dioxide.

The animal experiments were then repeated with additional steps. An esophageal temperature probe was utilized to monitor tissue temperature while intentionally creating lesions on the outer surface of the esophagus using an ablation catheter. Ablation of the esophageal wall was performed both with carbon dioxide insufflation and without carbon dioxide insufflation, to learn of the effects carbon dioxide has on the conduction of thermal energy.

In these evaluations, the esophagus was exposed through a large left thoracotomy. A multi-pole temperature probe was placed through the pig's mouth and down the esophagus under X-ray guidance. The ablation catheter was applied directly to the outer surface of the esophagus and the ablation electrode was aligned with one of the twelve (12) poles of the temperature sensor by X-ray. The ablation catheter was then energized. The measured temperature began to climb almost immediately, from a baseline temperature of 36.6 degrees C, reaching the critical 0.2 degrees C increase in less than five (5) seconds. With continued energy application, the temperature rose to 40 degrees C after thirty (30) seconds. The experiment was then repeated under the same conditions, with the only difference being carbon dioxide insufflation was added to the protocol as is explained in greater detail subsequently.

Prior to infusing carbon dioxide to test thermal insulation of the esophagus during ablation, an investigation into how long carbon dioxide would remain in place after injection into the peri-esophageal space was performed. After injecting 120 cc of carbon dioxide into the peri-esophageal fibro-fatty tissue, the tissue would instantly inflate with carbon dioxide, becoming considerably thicker. Yet, the tissue would gradually return to baseline geometry after approximately five (5) minutes. From this simple test it may be reasonably inferred that continuous insufflation with carbon dioxide would be preferable to insuring the insulating layer remained in place when needed during the ablation procedure.

Based on this observation, a 27-gauge needle attached to a long intravenous extension tube was attached directly to the regulator of a small tank of pressurized carbon dioxide. When the needle was inserted into the fibro-fatty tissue around the esophagus, it immediately inflated, as had been previously observed. But the cavity remained inflated until the supply of carbon dioxide was stopped. The rate of carbon dioxide delivery was arbitrarily titrated to be as low as possible with the regulator at hand.

When this experiment was repeated with an ablation catheter and a temperature probe (once again aligning the electrode with the temperature sensor under X-ray) and performing the ablation burn at the same power settings, the temperature readings were significantly different from those observed prior to infusion of carbon dioxide. After thirty (30) seconds of continuous burning, the temperature rose only 0.1 degrees C, from 36.6 degrees C to 36.7 degrees C, in contrast to the 3.4 degrees C observed when there was no carbon dioxide present; namely, 36.6 degrees to 40.0 degrees C. Accordingly, carbon dioxide injected into the fatty tissue surrounding the esophagus provided thermal insulation to the esophagus during such a procedure.

Dissection of the peri-esophageal tissue after only 120 cc of carbon dioxide injection or infusion reveals an 8 mm sheath or layer of emphysematous tissue that circumferentially surrounded the esophagus. This tissue is gas infused and conducts radio frequency energy and heat poorly. This 8 mm layer should push the posterior left atrium wall and the esophagus away from each other, thereby allowing aggressive burns to be created across the posterior left atrium wall without fear of esophageal injury.

A system for performing this procedure should preferably be simple for the electrophysiologist to utilize and not interfere with the underlying intra-cardiac catheter ablation procedure. The system should preferably remain in position during the ablation and cause no injury to the left atrium, the esophagus or any biological tissue. The system may also counter the effects of systemic carbon dioxide absorption by utilizing a feedback controller to deliver additional carbon dioxide as needed to maintain the required tissue separation. The system may include a temperature probe. Initially, doctors may place a temperature probe in the esophagus to ensure that the carbon dioxide infused tissue does create a thermal barrier. Once enough evidence exists that proves that the esophagus is thermally insulated, the temperature probe may not be needed. The system may also be utilized just once at the onset of the intra-cardiac catheter ablation procedure to achieve the desired separation between the esophagus and the left atrium and then subsequently removed to allow for the remainder of the ablation procedure, provided the effects of carbon dioxide absorption are negligible.

Such a system preferably comprises a reversibly deployable needle that advances a short distance from the end of a catheter and locks in that position, but that is in fixed geometric relationship to a sensor that allows its position to be identified on a mapping device such as CARTO® 3, and that has a mechanism for fixing the catheter in place, to prevent it from falling out during ablation. The system also comprises a valve, button, knob or any suitable device that connects the catheter to a small pressurized canister of carbon dioxide with a built-in regulator that: 1) controls the rate and volume of carbon dioxide that can be delivered over the course of the procedure; and 2) for safety, makes it impossible to accidentally hook the device to a gas other than carbon dioxide. The system may also comprise a custom sheath that allows the catheter to be inserted across the atrial septum and locked into position on the posterior left atrium wall, while providing a second lumen for the ablation catheter to be inserted into the left atrium for creation of the burns. Alternative exemplary embodiments are also contemplated as described in greater detail subsequently.

More specifically, an injection catheter for administering carbon dioxide through the left atrium wall as part of the above-described system preferably has certain attributes. The injection catheter should fit through a standard 8.5 French trans-septal sheath and have an integrated stop cock and syringe to allow sterile carbon dioxide to be drawn and delivered. In an alternative exemplary embodiment, the injection catheter may comprise an integral sterile carbon dioxide canister to decrease the setup time and make it easier to utilize. The injection catheter should preferably have the right handling characteristics and column strength to allow the needle to be advanced precisely at the desired point. In one exemplary embodiment, the needle assembly should preferably comprise a 27-gauge needle that only extends to the epicardium to ensure accurate carbon dioxide delivery. In an alternative exemplary embodiment, the 27-gauge needle may extend beyond the epicardium.

In an alternative exemplary embodiment, a catheter with balloons and a deployable needle that is placed through the pharynx and into the esophagus which allows injection of carbon dioxide into these same fibro-fatty tissues through the esophageal wall, in other words "inside out" from the esophageal lumen outward may be utilized. In this alternate exemplary embodiment, the catheter would be similar to the catheter described above.

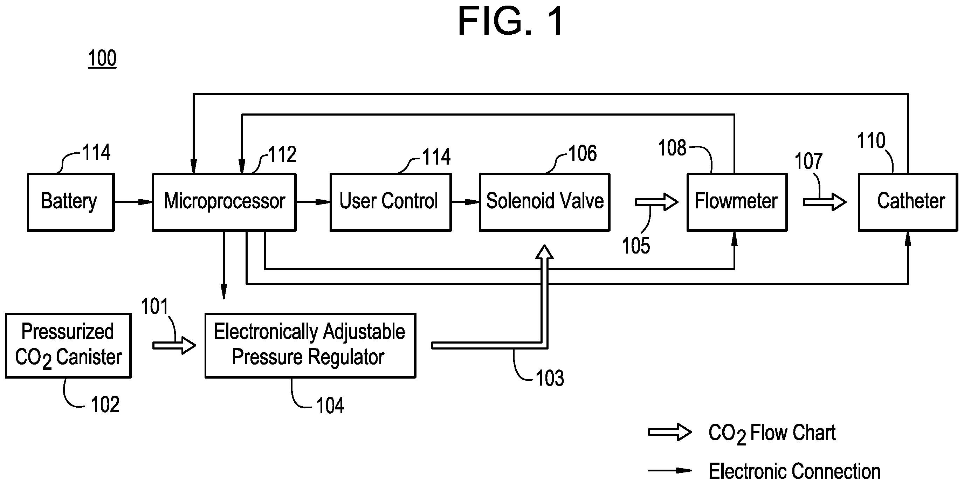

Referring now to Figure 1, there is illustrated an exemplary embodiment of a system 100 for the prevention of esophageal damage and/or fistula during intra-cardiac catheter ablation. The system 100 is configured to trans-septally deliver carbon dioxide through a minimally invasive catheter to create a gaseous pocket between the posterior wall of the left atrium and the esophagus. This pocket serves to thermally isolate and separate the esophagus from the left atrium during ablation to prevent esophageal damage or the formation of an atrial-esophageal fistula. It is important to note that the system 100 may be implemented utilizing a combination of discrete components, as a unitary, integrated system and/or a combination thereof.

The system 100 is configured as a closed-loop feedback control system and is illustrated in block diagram format for ease of explanation. Carbon dioxide, purified for use in biological applications, is supplied from a pressurized canister 102 and routed through a conduit 101 to a pressure regulator 104. As set forth above, special connectors may be utilized to prevent gas supplies other than carbon dioxide from being utilized. Although illustrated as a single discrete carbon dioxide canister, the gas may be supplied from any suitable source, for example, a central supply. In addition, the pressure regulator 104 may be connected directly to the pressurized canister 102. The pressure regulator 104 is electronically adjustable and is utilized to set and maintain the pressure at which the carbon dioxide is delivered. The operation of the pressure regulator 104 is the same as a pressure regulator on a SCUBA tank or home compressor. A pressure regulator simply maintains the pressure of the gas to be released at a set value for downstream use. The pressure regulator 104 is connected to a solenoid-controlled valve 106 through conduit 103. The solenoid-controlled valve 106 is utilized to control the flow rate of the carbon dioxide from the canister 102 or other supply. The solenoid-controlled valve 106 is connected to a flowmeter 108 via conduit 105. The flowmeter 108 measures the flow rate of the carbon dioxide exiting the solenoid-controlled valve 106 to ensure that it is at the desired flow rate for use in the procedure. The flowmeter 108 is connected to an injection catheter 110 through conduit 107. The injection catheter 110, which comprises a needle assembly described in greater detail subsequently, is utilized to precisely deliver the carbon dioxide to the desired location within the body as described herein. The conduits 101, 103, 105 and 107 may comprise any suitable material that does not react with carbon dioxide, for example, metallic materials such as stainless steel and polymeric materials such as polysiloxanes.

The system 100 also comprises a microprocessor or microcontroller 112. The microprocessor or microcontroller 112 is powered by a power supply 114. The power supply 114 may comprise a battery, either a primary battery or a secondary battery, and/or circuitry for converting power supplied from another source, for example, house power, into a voltage and current level suitable for the microprocessor 112 and other components of the system 100. The microprocessor 112 is programmed to output control signals to the flowmeter 108 and the catheter 110 based upon feedback signals from each as well as preprogrammed control parameters. The microprocessor 112 also outputs control signals to the pressure regulator 104 to adjust the pressure of the gas as required, and to a user control 114. The user control 114 is configured to allow the user of the system 100, for example, a physician or electrophysiologist, to set the parameters of operation via its connection to the solenoid-controlled valve 106 and operates as part of the feed-forward path of the control loop. The microprocessor 112, through its feedback control process automatically adjusts and maintains the operation of the system 100 in accordance with the user's settings. The microprocessor 112 may comprise any suitable processor and associated software and memory to implement the operation of the system 100.

It is important to note that all electronics and electrical connections are protected in a manner suitable for use in an operating or procedure theater. These precautions are necessary to prevent any interaction between an oxygen source and an electrical spark. In addition, all components are preferably manufactured for medical grade usage.

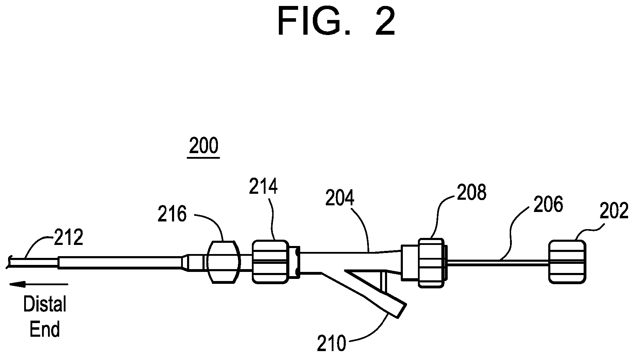

Referring now to Figure 2, there is illustrated a diagrammatic representation of the proximal region of an exemplary catheter that may be utilized for interventional procedures. The exemplary catheter comprises an elongate body having a proximal end and a distal end. The exemplary catheter 200 comprises a female luer lock connector 202 connected to a Tuohy Borst valve 204 via hypo tube 206 at the proximal valve end 208 of the Tuohy Borst valve 204. The carbon dioxide supply or pressurized cannister 102 illustrated in Figure 1 is connected to the injection catheter 200 via this connection point. As set forth above, unique connectors may be utilized to prevent connection to a different gas supply. In addition, this connection point 202 may be utilized to connect any suitable means for flushing the system. The Tuohy Borst valve 204 also comprises a Y-connection 210. The Y-connection 210 may be utilized to introduce fluids for any number of purposes, including the delivery of contrast agents for fluoroscopic visualization. A proximal shaft 212 is connected on one end to a luer connector 214 of the Tuohy Borst valve 204 via outer shaft luer hub 216 and on the other end to a distal shaft, not illustrated. The Tuohy Borst valve 204, the luer connector 214 and the outer shaft hub 216 are rotationally fixed together to work as a unitary structure. With this configuration, rotation of the Tuohy Borst valve 204 facilitates transmission of torque down the catheter shaft to a fixation coil, as described in detail subsequently, to engage and advance the fixation coil into the heart wall during a procedure. The distal end or region of the exemplary catheter 200 is continuous with the proximal end or region described herein; however, for ease of explanation as it relates to the present invention, the description and drawings are given independently. This basic catheter structure may be utilized for any number of interventional procedures, including the introduction and use of an injection catheter for the delivery of carbon dioxide. A detailed description of the Tuohy Borst valve and the proximal portion of the injection catheter, and the needle assembly or distal portion of the injection catheter of the present invention, as stated above, is given subsequently.

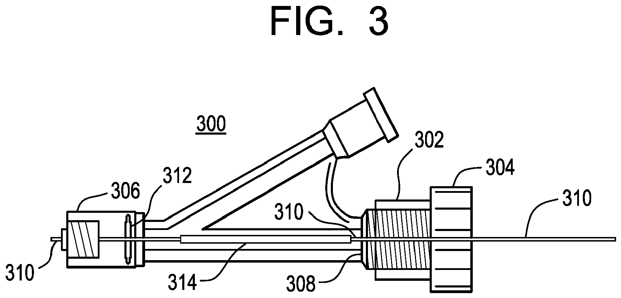

Figure 3 is a diagrammatic representation of a Tuohy Borst valve 300. The Tuohy Borst valve 300 is essentially the proximal region of the injection catheter. The Tuohy Borst valve 300 comprises a proximal valve 302 with screw lock 304. The proximal valve 302 is threaded to open and close the valve. The screw lock 304 of the proximal valve 302 may be turned clockwise to form a liquid tight seal around any instrumentation, for example, a hypo tube, introduced therethrough to provide a pneumostatic and hemostatic seal. The Tuohy Borst valve 300 also comprises a threaded luer connector 306 which connects the Tuohy Borst valve 300 to the shaft luer hub 216, illustrated in Figure 2, of the catheter as described briefly above and in greater detail subsequently.

A deformable O-ring 308 is positioned within the proximal valve 302 such that a hypo tube 310 portion of the injection catheter of the present invention passes therethrough. It is through this hypo tube 310 and ultimately a needle attached thereto that the carbon dioxide is introduced into the desired tissue. Referring back to Figure 1, the hypo tube 310 is part of the catheter block 110. As the screw lock 304 of the proximal valve 302 is tightened (clockwise for right-handed threads), the O-ring 308 is compressed which decreases its inside diameter. This action creates a compressive friction lock on the hypo tube 310 of the injection catheter, which may be utilized to lock the needle in place once the desired tissue depth is achieved. A second O-ring 312 is positioned within the threaded luer connector 306 to create a compressive seal for connection to the shaft luer hub 216, illustrated in Figure 2. The hypo tube 310 of the present invention comprises a stop mechanism 413. The stop mechanism 314 is a structure mounted around the hypo tube 310 in the region between the O-rings 308 and 312 of the Tuohy Borst valve 300. The diameter or size of the stop mechanism 314 is sufficient to prevent the hypo tube 310 from passing through either non-compressed O-ring 308, 312 to prevent accidental over-deployment of the needle into the target tissue as well as unintentional over-retraction of the injection catheter from the introducer or trans-septal sheath as described in greater detail herein. The hypo tube 310 may comprise any suitable biocompatible material, including any hypo tube materials currently in use in catheters. The stop mechanism 314 may also comprise any suitable biocompatible material. In the exemplary embodiment, the stop mechanism 314 comprises a polymeric material and is bonded to the hypo tube 310 utilizing any suitable means including welding and adhesives.

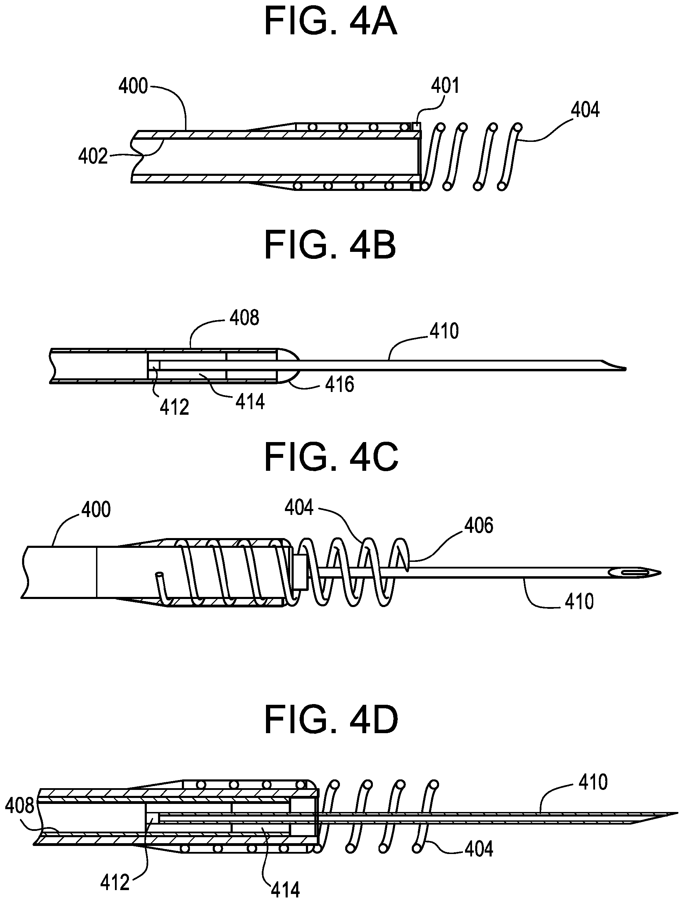

Figures 4A - 4D are diagrammatic representations of the distal portion of an exemplary injection catheter Figure 4A is a sectional or cutaway view of the distal region of an outer sheath 400 of the injection catheter. The outer sheath 400 comprises a tubular structure 402 in which an inner sheath 408 is coaxially positioned. Attached to the distal end of the outer sheath 400 is a fixation coil 404. The fixation coil 404 comprises a corkscrew configuration that functions as a reversible anchoring system to secure the injection catheter in place during carbon dioxide insufflation. The fixation coil 404 is affixed to the outer surface of the distal end of the tubular structure 402 such that a first portion thereof is sealed to the tubular structure 402 and a second portion thereof extends past the end of tubular structure for anchoring in the myocardium. To ensure that the fixation coil 404 and the tubular structure 402 move and operate as a unitary structure, the fixation coil 404 is permanently bonded to the tubular structure 402 by any suitable means. In one exemplary embodiment, a UV curable adhesive is utilized. The tip of the fixation coil 404 comprises a sharp point 406, illustrated in Figure 4D, to easily pierce the cardiac tissue. The tubular structure 402 may comprise any suitably rigid, biocompatible material that may be navigated through a tortuous vasculature. Standard catheter materials may be utilized. The fixation coil 404 may comprise and suitable rigid biocompatible material that can be twisted into cardiac tissue and anchor therein. Metallic material, for example, stainless steel, or polymeric materials may be utilized. In the exemplary embodiment, the fixation coil comprises stainless steel. The most distal end of the outer sheath 400 also features a radiopaque marker or band 401 for fluoroscopic visualization. The radiopaque marker 401 may be formed from any suitable material, for example, tantalum and bonded to the outer sheath 400 utilizing any suitable means. The radiopaque marker 401 is positioned on the outer surface of the outer sheath 400 and bonded within the inside diameter of the fixation coil 404.

In operation and prior to needle 410, illustrated in Figure 4B, deployment, the injection catheter is navigated into a position proximate the left atrium such that the fixation coil 404 may be screwed into the myocardium of the left atrium in order to maintain catheter position during carbon dioxide insufflation. Essentially, the fixation coil 404 reversibly anchors the outer sheath 400 to the patient's heart by a simple twisting motion of the injection catheter. As set forth above, twisting or rotation of the Tuohy Borst valve 204, Figure 2, by the physician facilitates transmission of torque through the outer sheath 400.

Figure 4B is a sectional or cutaway view of the distal region of the inner sheath 408. The inner sheath 408 comprises a needle 410 that may be advanced through the posterior wall of the left atrium into the juxta-esophageal space or fibro-fatty tissue to deliver a controlled dose of carbon dioxide during an intra-cardiac catheter ablation procedure and then removed. The outer sheath 400 prevents the needle 410 from contacting the surrounding vasculature during navigation of the injection catheter to the target insufflation site. The needle 410 may be retracted back into the outer sheath 400 upon completion of the procedure. The needle 410, which may comprise any suitable material and be sized as set forth herein is connected to the hypo tube 310, illustrated in Figure 3, via a plastic extrusion 412. In the exemplary embodiment, the needle 410 comprises surgical steel, may comprise any other suitable metallic materials, including nitinol and highly radiopaque materials in alternate embodiments. The connection of the needle 410 to the plastic extrusion 412 and the connection of the plastic extrusion 412 to the hypo tube 310 may be made by any suitable means such that an unobstructed flow of carbon dioxide may be achieved while allowing the three components to act as a unitary structure. In operation, the needle 410 is advanced and retracted by movement of the hypo tube 310 within the outer sheath 400 which is anchored in the myocardium. The needle 410 and the plastic extrusion 412 are coaxially positioned within the lumen of the braided shaft forming the inner sheath 408 inside of a centering element 414. The needle 410 is also slidably sealed to the tip of the inner sheath 408. The centering element 414 and the seal 416 may be made of any suitable biocompatible material.

Figure 4C illustrates the assembled injection catheter distal portion with the inner sheath positioned within the outer sheath 400 and the needle 410 extending therefrom. Figure 4D illustrates a sectional or cutaway view of the assembled injection catheter distal portion. As explained herein, the injection catheter is navigated through the vasculature and into position proximate the left atrial myocardium. The injection catheter is anchored into position utilizing the fixation coil 404. When properly anchored, the needle 410 is advanced via the hypo tube 310 at the proximal end until the desired tissue depth is reached. Carbon dioxide is then injected into the fibro-fatty tissue that separates the posterior left atrium wall from the esophagus via the exemplary system illustrated in Figure 1 until the ablation procedure is completed. Upon completion, the fixation coil 404 is removed by twisting the outer sheath 400 in the opposite direction for insertion, and the injection catheter may be removed. The injection catheter does not in any way interfere with the left atrium ablation procedure. In addition, it is important to note that the injection catheter of the present invention may be introduced via the same introducer or trans-septal sheath as the ablation catheter or a completely different one.

Although the distal portion and the proximal portion of the injection catheter is shown in different illustrations for ease of explanation, the two portions form a continuous structure with inner and outer sheaths.

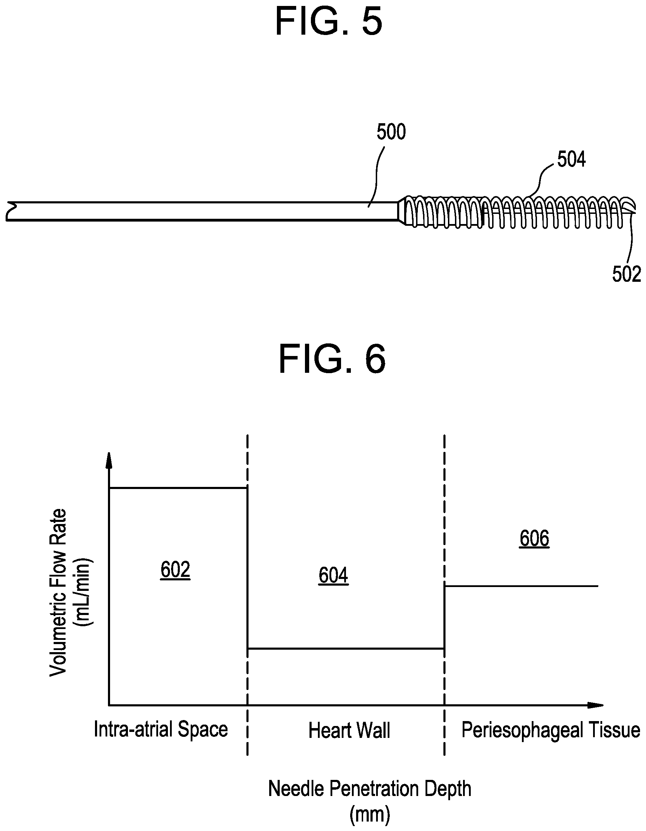

In an alternate exemplary embodiment, the needle may be fixed in place with the fixation coil. Wherein with the exemplary embodiment described above the needle moves independently of the fixation coil by means of the longitudinal movement of the hypo tube, in this alternate exemplary embodiment, the needle remains fixed relative to the coil. In other words, as the fixation coil is twisted into and out of the cardiac tissue, the needle advances or retracts accordingly. Figure 5 illustrates this alternate exemplary embodiment. As illustrated, the needle 502 is positioned within the fixation coil 504 and both are attached to the end of an outer sheath 500 with the needle 502 in fluid communication as described above.

As set forth above, the needle-tipped catheter or injection catheter is advanced through the posterior wall of the left atrium into the fibro-fatty tissue or juxta-esophageal space to deliver a controlled dose of carbon dioxide to expand the tissue and create an insulation layer during an ablation procedure. In the preferred embodiment, the delivery of carbon dioxide is continuous during ablation rather than through discrete delivery so as to safely maintain tissue expansion. Upon completion of the procedure, the needle may be retracted into the outer sheath of the catheter. In order to precisely deliver the carbon dioxide, the system may employ one or more methodologies to determine the deployment depth of the needle without the need for direct visual confirmation. It is important to note that visual confirmation would be a viable alternative but involve additional complexities.

In one exemplary method, the flow rate of the carbon dioxide exiting the needle may be monitored to determine the resistance to flow. The left atrium space, the myocardial tissue and the fibro-fatty tissue all have different resistivity to gas flow. Accordingly, the physician may simply determine in which tissue layer the needle tip resides by referencing a tissue layer flow rate characterization chart. In an alternative embodiment, the microprocessor 112 (Figure 1) may be programmed with the flow resistivity of the various tissues or media in the body and receiving feedback from the flowmeter 108 as to the flow rate exiting from the needle, automatically generate an alert via some suitable signal to be displayed or an audible signal that indicates that the proper location for the needle has been achieved. The flowmeter may be utilized to measure the flow resistance at the needle tip and provide feedback directly to the physician or through the microprocessor 112 rather than flowmeter 108.

Figure 6 is a graphical representation of the relative flow rates of carbon dioxide in the different regions/tissues. The vertical axis represents volumetric flow rate and the horizontal axis represents needle penetration depth in mm. In the first region 602 which represents the intra-atrial space, the flow rate of carbon dioxide is high relative to the other regions as one may expect. In the second region 604 which represents the heart wall, the flow rate of carbon dioxide is significantly lower that the first region 602 given the density of the cardiac tissue. In the third region 606 representing the periesophageal tissue, the flow rate of carbon dioxide is higher than in the heart wall due to lower tissue density but lower than in the intra-atrial space. By measuring flow rate as the needle progresses, one may determine needle location.

In another exemplary method, the electrical activity of the tissue in which the needle is positioned may be monitored. The myocardium has a distinctly different electrical activity profile than the left atrium space and the surrounding tissue. By monitoring this activity with the needle tip, the physician can determine the point at which the needle has contacted and subsequently passed through the myocardium and entered into the fibro-fatty tissue. In this exemplary embodiment, the needle may be configured to provide feedback to a stand-alone sensing circuit or one that is part of the microprocessor. The sensing circuit may be configured to measure the electrical activity, for example, voltage/potential and/or resistance/impedance. As in the previously described embodiment, this information may be routed through the microprocessor 112 which will automatically make the determination or to any suitable device for altering the physician.

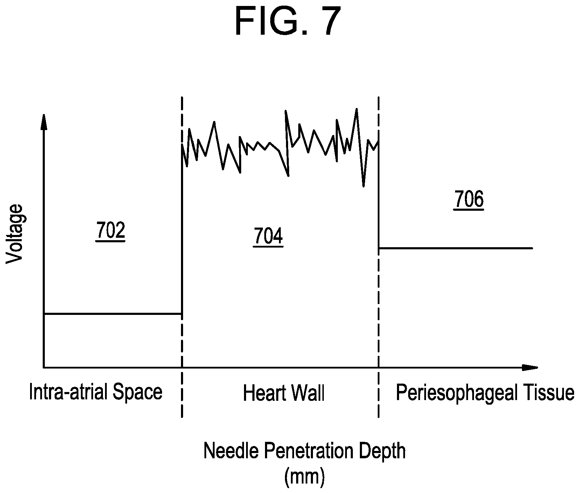

Figure 7 is a graphical representation of voltage, or potential, vertical axis, versus needle penetration depth, horizontal axis. As illustrated, in the first region 702 corresponding to the intra-atrial space, the voltage sensed by the needle is steady-state and low. In the second region 704 corresponding to the heart wall, the electrical activity is not steady-state and at a higher potential than the first region. In the third region 706 corresponding to the periesophageal tissue, the electrical activity is once again steady-state and of lower potential than the cardiac tissue of the heart wall.

In both exemplary embodiments, real-time monitoring of needle location is achieved without the need for direct visualization.

In accordance with an alternative approach as briefly set forth above, an alternate device placed into the esophagus consists of a catheter with a balloon and a deployable needle and may be used to inject carbon dioxide into the fibro-fatty tissues between the esophagus and the heart. This device, hereinafter will be referred to as a transesophageal catheter, and its various components may be introduced into the esophagus via a variety of methods including an endotracheal device or through a nasogastric tube. In one exemplary embodiment, the device 110 (illustrated in Figure 1) is configured for straight deployment of the needle proximal to the balloon. In a second exemplary embodiment, the device 110 is configured for needle deployment distal to the balloon, wherein the needle is deviated laterally via a curved through hole residing in a component located at the tip of the device. In each of these embodiments, a balloon is utilized to anchor the device in position rather than a fixation coil as described above. The compliant nature of the balloon allows the user to inflate the balloon until it reaches the equivalent internal diameter of the esophagus, thereby making the device agnostic to variations in esophageal anatomy (esophageal diameter, curvature, longitudinal variation, and the like). Other non-compliant balloon materials may be used to be precisely sized to the patient's esophageal anatomy. Balloon inflation and deflation is accomplished utilizing techniques known in the catheter art. Regardless of the configuration, the device allows a physician to deliver carbon dioxide through a minimally invasive catheter to create a gaseous pocket between the posterior wall of the left atrium and the esophagus. This pocket serves to thermally insulate and separate the esophagus from the left atrium during ablation to prevent esophageal damage and/or the formation of an atrioesophageal fistula. In these embodiments, the catheter is configured differently, but its connection to and operation is the same as in the above-described embodiments.

In accordance with the first exemplary embodiment of the transesophageal catheter, the device comprises two bonded catheters; herein referred to as a needle catheter and a balloon catheter. The needle catheter resides within a protective sheath that functions to prevent the needle point at the termination of the needle catheter from damaging the esophagus during placement. The balloon catheter comprises a flexible catheter shaft with high-torque reinforced braiding to aid in the placement of the device at the target insufflation site within the esophagus. Furthermore, the high-torque braiding in the balloon catheter provides the user with precise control of the rotational positioning of the device within the esophagus, thereby allowing for precise aiming of the needle to the lateral aspect of the esophagus. The balloon catheter comprises an inflatable balloon bonded to a flexible catheter and with an inflation side-port located at the proximal end of the catheter. The balloon functions to secure the catheter in place during insufflation. In addition, the balloon may function to bias the injection needle to a more lateral engagement with the esophageal lumen. As set forth briefly above, in this embodiment, the needle at the terminal aspect of the needle catheter engages with the esophageal lumen proximal to the inflated balloon. The transesophageal catheter may also comprise a three-dimensional positional tracking device to locate the catheter tip in space relative to the surrounding anatomy as well as to measure insufflation. This device may preferably be compatible with the Carto®3 System. It is important to note that all design aspect of the carbon dioxide delivery system as well as means for tracking position and flow rates are applicable to this exemplary embodiment.

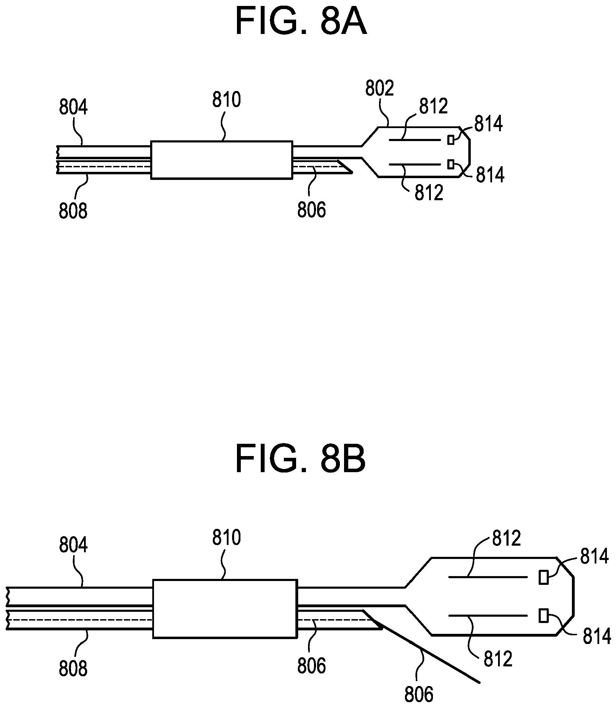

Referring now to Figures 8A and 8B, there is illustrated a first exemplary transesophageal catheter 800. The transesophageal catheter 800 is connected to the proximal shaft 212, see Figure 2. In Figure 8A the balloon 802 is attached to the balloon catheter 804 and is illustrated in its non-inflated state and the needle 806, illustrated in phantom, is positioned wholly within the outer sheath of the needle catheter 808 or in the non-deployed state. As set forth above, the balloon catheter 804 and the needle catheter 808 are bonded together. In alternative embodiments, a single catheter with two lumens may be utilized. In the illustrated embodiment, the two catheters 804 and 808 are bonded together via a band 810, but may be joined in any suitable manner, including adhesives. The balloon 802 may comprise any suitable balloon. In more preferred embodiments, the balloon 802 may comprise one or more anti-slip grips 812 on its external surface in the form of ribs, spikes, pyramids, bumps, villi or similar protrusions. In addition, one or more radiopaque markers or materials 814, for example, barium sulfate or radiopaque metal markers, may be attached to or embedded within the balloon 802 to aid the user in the orientation of the device to the patient's anatomy to aim the needle 806. The balloon 802 reversibly secures the transesophageal catheter 800 in position within the esophagus during the procedure. In other words, when the balloon 802 is not inflated, the catheter 800 may be position and/or removed after the procedure. However, when the balloon 802 is inflated, Figure 8B, the catheter is secured in position and may not be removed. The needle 806 is a 25-gauge needle that is advanceable through the needle catheter 808 through the lumen of the esophagus into the fibro-fatty tissue lining the longitudinal aspect of the esophagus space to deliver a controlled dose of carbon dioxide. Figure 8B illustrates the needle 806 deployed from the catheter 808. The needle 806 may be safely retracted into the catheter after insufflation is complete. As in the other embodiments, the physician is guided to the proper needle depth and the flow rates of carbon dioxide controlled through the means disclosed herein.

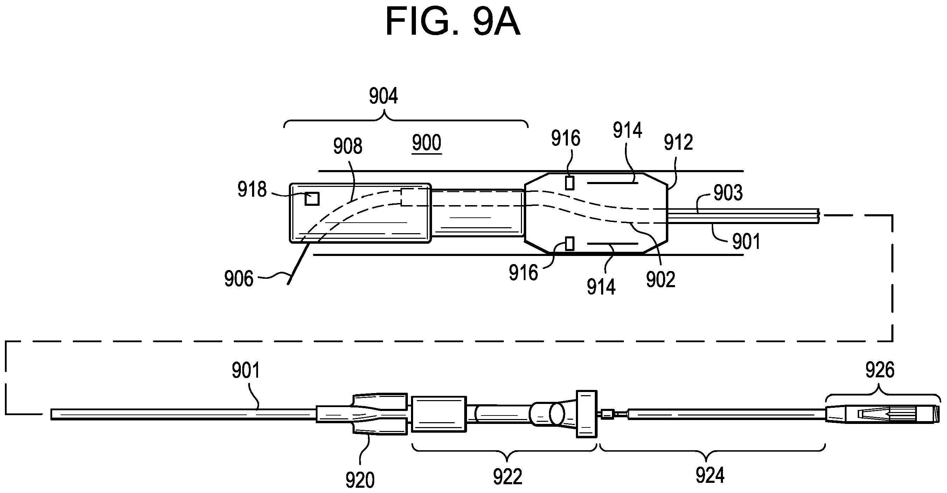

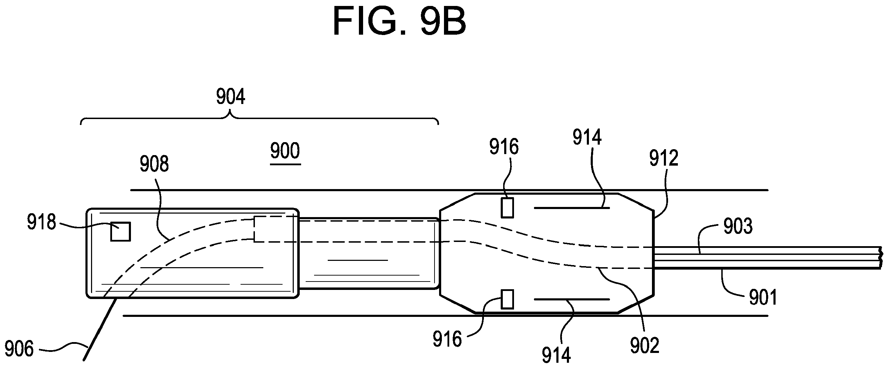

Referring now to Figures 9A and 9B, there is illustrated a second exemplary transesophageal catheter 900. The second exemplary catheter comprises three coaxially nested catheters; namely, an outer sheath, a needle catheter and a balloon catheter. The outer sheath comprises a flexible catheter shaft with high-torque reinforced braiding to aid in the placement of the device at the target insufflation site within the esophagus. Furthermore, the high-torque braiding in the balloon catheter provides the user with precise control of the rotational positioning of the device within the esophagus, thereby allowing for precise aiming of the needle to the lateral aspect of the esophagus. The balloon catheter resides within the interior lumen of the outer sheath and comprises an inflatable balloon bonded to a flexible catheter and with an inflation side-port located at the proximal end of the catheter. The balloon functions to secure the catheter in place during insufflation. The needle catheter resides within the interior lumen of the balloon catheter and serves as the pathway through which carbon dioxide gas will flow through the needle at the termination of the needle catheter. As set forth briefly above, in this embodiment, the needle at the terminal aspect of the needle catheter engages with the esophageal lumen proximal to the inflated balloon. The transesophageal catheter may also comprise a three-dimensional positional tracking device to locate the catheter tip in space relative to the surrounding anatomy as well as to measure insufflation. Positioning or tracking of the transesophageal catheter may be done in more than one way. For example, in one embodiment, it may be determined if the position of the catheter itself changes. Alternatively, a secondary catheter positioned within the heart may be utilized to measure the space between the left atrium and the esophagus using two sensors. In either embodiment the device may preferably be compatible with the Carto®3 System. It is important to note that all design aspect of the carbon dioxide delivery system as well as means for tracking position and flow rates are applicable to this exemplary embodiment.

In Figure 9A, the transesophageal catheter 900 comprises the distal aspect of the aforementioned balloon catheter as it exits the outer sheath 905. A coupling component 902 is utilized to connect the balloon catheter 901 to a needle biasing component 904. The needle catheter 903 within the interior of the balloon catheter lumen extends from the carbon dioxide inlet port at the utmost proximal end of the device and terminates within the needle biasing component 904. The needle biasing component 904 functions to curve or bias the needle 906 at a lateral angle that allows for proper entrance into the fibro-fatty tissue within the periesophageal space. Furthermore, the needle biasing component 904 serves as a protective sheath in which resides the needle in the undeployed state to prevent damage of the esophagus during placement of the device. The carbon dioxide inlet port of the needle catheter 903 is attached to a user-controlled plunger mechanism or other suitable means to advance the needle, including the hypo tube 310 (Figure 3), and when the plunger is depressed by the user, the needle catheter 903 is equally translated within the lumen of the balloon catheter 901, thereby deploying the needle through the curved through hole of the needle biasing component. Figure 9B illustrates a detailed, section of the needle biasing component 904. The needle biasing component 904 includes a conduit 908 through which the needle 906 is advanced to an exit port 910. The needle biasing component 904 may comprise any suitable configuration and is preferably substantially cylindrical in shape. Using a more lubricious material in the construction of the needle biasing component would allow for more aggressive curvature and thus a small diameter needle biasing component 904. In the exemplary embodiment, the needle biasing component 904 comprises PolyJet, which is a standard three-dimensional printing material, but may comprise any suitable biocompatible and lubricious material so that the needle 906 may easily pass therethrough. The balloon 912 is mounted to the transesophageal catheter 900 proximal to the physician so that deployment of the needle 906 is distal relative to the balloon 912 as set forth above. The balloon 912 may also comprise anti-slip grips 914 on its external surface in the form of ribs, spikes, pyramids, bumps, villi or similar protrusions. In addition, one or more radiopaque markers or other radiopaque materials 916 may be attached to or embedded within the balloon 912 to aid the user in orienting the device within the body. The needle 906 and the balloon 912 are the same as in the above described exemplary embodiment. In addition, deployment and retraction of the needle is accomplished via the mechanisms described herein.

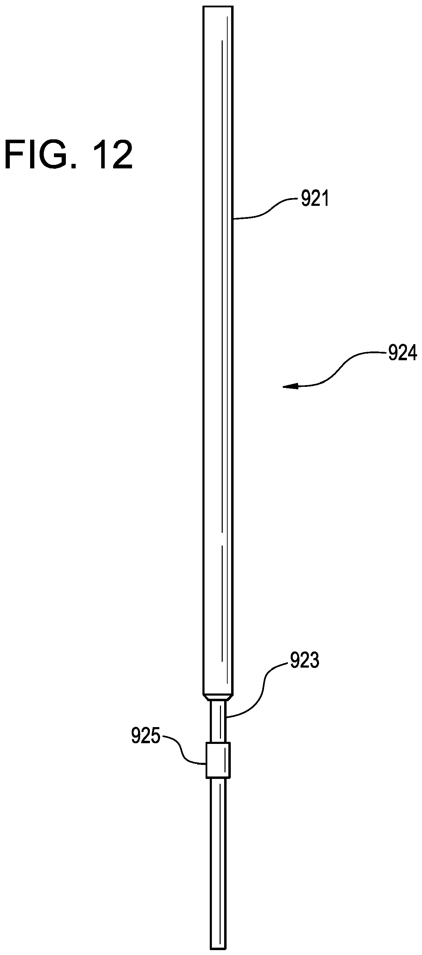

Viewing the proximal end of the transesophageal catheter 900, the balloon catheter 901 is connected to a luer adapter 920 which is connected to a Tuohy Borst valve/assembly 922 which is connected to a slider mechanism 924 which is connected to a proximal luer connector 926 which is connected to the carbon dioxide supply described herein. The slider mechanism 924 is utilized for needle 906 advancement and is described in greater detail with respect to Figure 12 below.

In the previously described embodiments, various position sensing methodologies are disclosed. In the transesophageal catheter embodiments, a three-dimensional position sensor 918 may be positioned within the needle biasing component 904 as illustrated in Figure 9B. This sensor 918 may be utilized to locate the tip of the transesophageal catheter in space relative to the surrounding anatomy as well as to measure insufflation. By working in combination with the Biosense Webster CARTO® 3 system, the distance between the sensor/probe in the heart and the sensor 918 in the catheter tip can be measure and monitored to determine changes in position thereby indicating the amount of insufflation. These as well as all other calculations are done via the microprocessor 112 of the overall system shown and described with respect to Figure 1. The CARTO® 3 system, particularly its navigation features, are set forth in United States Patent Numbers 6,400,981; 6,650,927; 6,690,963; 6,716,166; 6,788,967; 7,090,639; 7,517,318; 7,536,218; 7,604,601; 7,681,579; 7,684,850; 7,735,349; 7,756,576; 7,831,076; 7,848,787; 7,848,789; 7,869,865; 8,075,486; 8,320,711; 8,359,092; 8,456,182; 8,478,379; 8,478,383; 8,532,738; 8,676,305; 8,870,779; 9,023,027; 9,460,563; 9,498, 147.

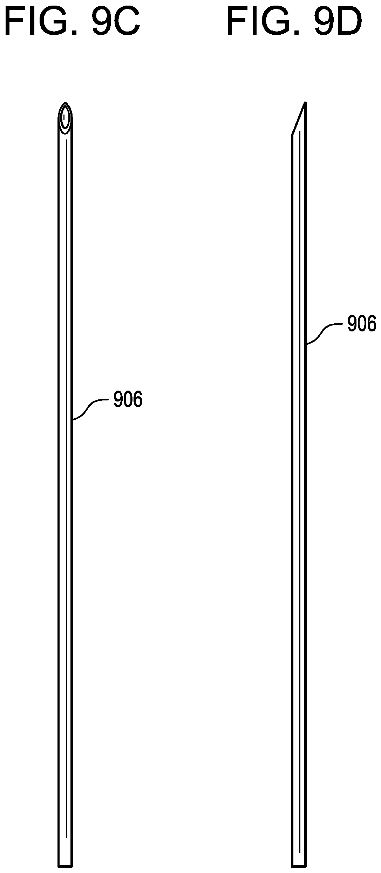

The needle in any of the exemplary embodiments set forth herein, including those by which the device is puncturing through the esophagus is made of nitinol alloy in this exemplary embodiment, however, a variety of other materials may be used as well. Preferably, the needle is non-ferrous in nature. Furthermore, coatings applied to the needle may be used to increase its lubricity, for example, polytetrafluoroethylene or PTFE, to aid in esophageal puncture and/or with antibacterial agents to prevent infection. Anti-adhesive surface coatings using concepts of surface chemistry and functionality including ions and polymer coats may be used. The needle surface may be coated with bactericidal substances such as Chitosan-vancomycin and silver. Nanotopographic surface modifications may also be used as either anti-adhesives or bactericidal features. Furthermore, a radiopaque plating (for example, gold or platinum) can be applied to the needle to aid in fluoroscopic visualization of the needle. An exemplary needle 906 is illustrated in Figures 9C and 9D. As set forth above, the slider mechanism 924 is utilized for needle 906 advancement or retraction. Referring to Figure 12, the slider mechanism comprises a translation sleeve 921 which is connected to the needle 906, a translation rail 923 upon which the translation sleeve 921 travels, and a stop 925 which prevents over deployment of the needle 906. The translation rail 923 is connected at its proximal end to the proximal luer connector 926.

It is also important to note that the balloon utilized in the above-described exemplary embodiments may comprise any suitable type of catheter delivered balloon and are both inflated and deflated in the standard manner.

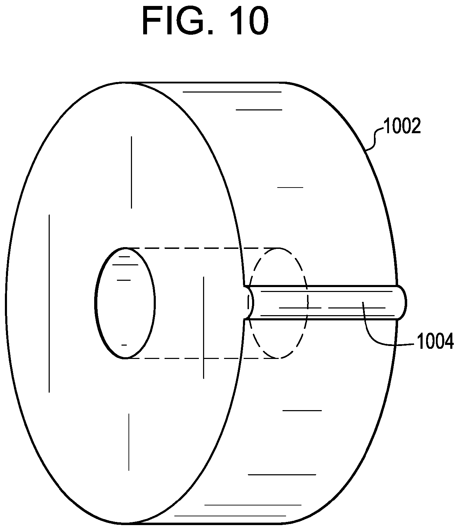

The device may also possess "clocking" indicators on the handle aspect of the device to guide the user in catheter rotation orientation and position to aid in correctly advancing the needle. Figure 10 is a diagrammatic representation of such an indicator system. As illustrated, an indicator wheel 1002 comprises a raised edge 1004 or other suitable demarcation to provide both a tactile and visual marker to guide the physician in needle deployment. The indicator wheel 1002 would form part of the handle assembly of the Tuohy Borst valve 300 illustrated in Figure 3. The indicator wheel 1002 would be attached to the catheter in such a way as to indicate the advancement or retraction of the needle. For example, the indicator wheel 1002 may be attached to the hypo tube 310 proximate the threaded luer connector 306. Any suitable arrangement that preferably provides both a tactile and visual marker for the needle deployment vector may be utilized.

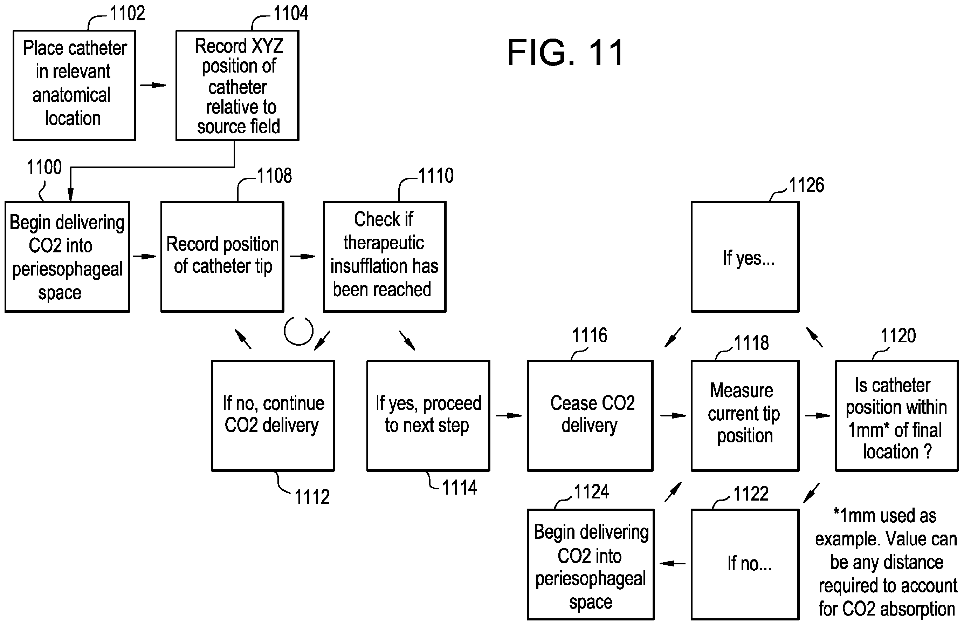

Referring now to Figure 11, there is illustrated a simple flow diagram of the overall process. In step 1102, the physician places the catheter in the desired anatomical location, for example, the esophagus proximate the left atrium of the heart. Once the catheter is in position, the coordinates, in three-dimensional space, of the catheter is recorded relative to the source field, step 1104. Once these initial coordinates are recorded, carbon dioxide is delivered into the periesophageal space, step 1106. In step 1108, the position of the catheter tip is recorded, once again in three-dimensional space. At step 1110, a check is performed to see if therapeutic insufflation has been achieved. If therapeutic insufflation has not been achieved, additional carbon dioxide is delivered, step 1112. If therapeutic insufflation has been achieved, step 1114, then the delivery of carbon dioxide is stopped, step 116. At step 1118, a measurement of the catheter tip in three-dimensional space is repeated. At step 1120, a determination is made as to whether the catheter tip is within a certain distance from the final desired position, for example, 1 mm. The determination may be made as previously described. This value is utilized as an example. The actual value may be any distance required to account for carbon dioxide absorption. If the catheter tip is not within a predetermined distance from the final desired position, step 1122, then proceed to step 1124 wherein additional carbon dioxide is delivered into the periesophageal space wherein step 1118 is repeated. If the catheter tip is within the predetermined position, step 1126, carbon dioxide delivery is stopped, step 1116. The logic and calculation are performed via the microprocessor 112 of the system. Although shown and described in what is believed to be the most practical and preferred embodiments, it is apparent that departures from specific designs described and shown will suggest themselves to those skilled in the art and may be used without departing from the scope of the invention. The present invention is not restricted to the particular constructions described and illustrated but should be constructed to cohere with all modifications that may fall within the scope of the appended claims.

## Claims

1. A catheter-based system (100) for the delivery of carbon dioxide to the fibro-fatty tissue between the esophagus and the heart for the prevention of esophageal damage and/or fistula during intra-cardiac ablation of the left atrium, the system comprising:
a transesophageal catheter (110) configured for introduction through the esophagus of a patient, the transesophageal catheter including a needle delivery device and associated needle (410) and an anchoring mechanism;
a gas supply (102) configured to deliver gas to the needle through the transesophageal catheter, the gas supply being connected at the proximal end of the transesophageal catheter; and
a feedback control system configured to regulate the pressure and flow rate of the gas from the gas supply to the needle.

2. The catheter-based system for the delivery of carbon dioxide delivery to the fibro-fatty tissue between the esophagus and the heart for the prevention of esophageal damage and/or fistula during intra-cardiac ablation of the left atrium according to Claim 1, wherein the needle delivery device is configured to deploy and retract the needle into and from the fibro-fatty tissue through the wall of the esophagus.

3. The catheter-based system for the delivery of carbon dioxide delivery to the fibro-fatty tissue between the esophagus and the heart for the prevention of esophageal damage and/or fistula during intra-cardiac ablation of the left atrium according to Claim 2, wherein the anchoring mechanism secures the transesophageal catheter in position within the esophagus.

4. The catheter-based system for the delivery of carbon dioxide delivery to the fibro-fatty tissue between the esophagus and the heart for the prevention of esophageal damage and/or fistula during intra-cardiac ablation of the left atrium according to Claim 3, wherein the anchoring mechanism comprises a balloon (802) deployable within the esophagus.

5. The catheter-based system for the delivery of carbon dioxide delivery to the fibro-fatty tissue between the esophagus and the heart for the prevention of esophageal damage and/or fistula during intra-cardiac ablation of the left atrium according to Claim 4, wherein the needle is deployable proximal to the balloon.

6. The catheter-based system for the delivery of carbon dioxide delivery to the fibro-fatty tissue between the esophagus and the heart for the prevention of esophageal damage and/or fistula during intra-cardiac ablation of the left atrium according to Claim 4, further comprising a needle biasing component (904).

7. The catheter-based system for the delivery of carbon dioxide delivery to the fibro-fatty tissue between the esophagus and the heart for the prevention of esophageal damage and/or fistula during intra-cardiac ablation of the left atrium according to Claim 6, wherein the needle is deployable distal to the balloon via the needle biasing component.

8. The catheter-based system for the delivery of carbon dioxide delivery to the fibro-fatty tissue between the esophagus and the heart for the prevention of esophageal damage and/or fistula during intra-cardiac ablation of the left atrium according to Claim 4, further comprising a means for determining needle position and insufflation.

9. The catheter-based system for the delivery of carbon dioxide delivery to the fibro-fatty tissue between the esophagus and the heart for the prevention of esophageal damage and/or fistula during intra-cardiac ablation of the left atrium according to Claim 8, wherein the means for determining needle position and insufflation comprises a three-dimensional position sensor (918).

## Patentansprüche

1. Katheterbasiertes System (100) zur Abgabe von Kohlendioxid an das Fibrofettgewebe zwischen dem Ösophagus und dem Herzen zur Verhinderung von Ösophagusschäden und/oder Fistelbildung während der intrakardialen Ablation des linken Vorhofs, wobei das System umfasst:
einen transösophagealen Katheter (110), der zur Einbringung durch den Ösophagus eines Patienten ausgestaltet ist, wobei der transösophageale Katheter eine Nadelabgabevorrichtung und die dazugehörige Nadel (410) und einen Verankerungsmechanismus einschließt;
eine Gaszufuhr (102), die ausgestaltet ist, um der Nadel durch den transösophagealen Katheter hindurch Gas zuzuführen, wobei die Gaszufuhr an das proximale Ende des transösophagealen Katheters angeschlossen ist; und
ein Rückmeldesteuersystem, das ausgestaltet ist, um den Druck und die Flussrate des Gases von der Gaszufuhr zu der Nadel zu regeln.

2. Katheterbasiertes System zur Abgabe von Kohlendioxidabgabe an das Fibrofettgewebe zwischen dem Ösophagus und dem Herzen zur Verhinderung von Ösophagusschäden und/oder Fistelbildung während der intrakardialen Ablation des linken Vorhofs nach Anspruch 1, wobei die Nadelabgabevorrichtung ausgestaltet ist, um die Nadel durch die Ösophaguswand hindurch in das Fibrofettgewebe hinein auszubringen und aus diesem zurückzuziehen.

3. Katheterbasiertes System zur Abgabe von Kohlendioxidabgabe an das Fibrofettgewebe zwischen dem Ösophagus und dem Herzen zur Verhinderung von Ösophagusschäden und/oder Fistelbildung während der intrakardialen Ablation des linken Vorhofs nach Anspruch 2, wobei der Verankerungsmechanismus den transösophagealen Katheter innerhalb des Ösophagus in Position sichert.

4. Katheterbasiertes System zur Abgabe von Kohlendioxidabgabe an das Fibrofettgewebe zwischen dem Ösophagus und dem Herzen zur Verhinderung von Ösophagusschäden und/oder Fistelbildung während der intrakardialen Ablation des linken Vorhofs nach Anspruch 3, wobei der Verankerungsmechanismus einen Ballon (802) umfasst, der innerhalb des Ösophagus ausbringbar ist.

5. Katheterbasiertes System zur Abgabe von Kohlendioxidabgabe an das Fibrofettgewebe zwischen dem Ösophagus und dem Herzen zur Verhinderung von Ösophagusschäden und/oder Fistelbildung während der intrakardialen Ablation des linken Vorhofs nach Anspruch 4, wobei die Nadel proximal zu dem Ballon ausbringbar ist.

6. Katheterbasiertes System zur Abgabe von Kohlendioxidabgabe an das Fibrofettgewebe zwischen dem Ösophagus und dem Herzen zur Verhinderung von Ösophagusschäden und/oder Fistelbildung während der intrakardialen Ablation des linken Vorhofs nach Anspruch 4, des Weiteren umfassend eine Nadelvorspannkomponente (904).

7. Katheterbasiertes System zur Abgabe von Kohlendioxidabgabe an das Fibrofettgewebe zwischen dem Ösophagus und dem Herzen zur Verhinderung von Ösophagusschäden und/oder Fistelbildung während der intrakardialen Ablation des linken Vorhofs nach Anspruch 6, wobei die Nadel distal zu dem Ballon über die Nadelvorspannkomponente ausbringbar ist.

8. Katheterbasiertes System zur Abgabe von Kohlendioxidabgabe an das Fibrofettgewebe zwischen dem Ösophagus und dem Herzen zur Verhinderung von Ösophagusschäden und/oder Fistelbildung während der intrakardialen Ablation des linken Vorhofs nach Anspruch 4, des Weiteren umfassend ein Mittel zur Bestimmung von Nadelposition und Insufflation.

9. Katheterbasiertes System zur Abgabe von Kohlendioxidabgabe an das Fibrofettgewebe zwischen dem Ösophagus und dem Herzen zur Verhinderung von Ösophagusschäden und/oder Fistelbildung während der intrakardialen Ablation des linken Vorhofs nach Anspruch 8, wobei das Mittel zum Bestimmen von Nadelposition und Insufflation einen dreidimensionalen Positionssensor (918) umfasst.

## Revendications

1. Système à base de cathéter (100) pour l'administration de dioxyde de carbone au tissu fibro-graisseux entre l'œsophage et le cœur pour la prévention de dommages œsophagiens et/ou de fistules pendant une ablation intra-cardiaque de l'oreillette gauche, le système comprenant :
un cathéter transœsophagien (110) configuré pour être introduit à travers l'œsophage d'un patient, le cathéter transœsophagien comprenant un dispositif de délivrance d'aiguille et une aiguille associée (410) et un mécanisme d'ancrage ;
une alimentation en gaz (102) configurée pour délivrer du gaz à l'aiguille à travers le cathéter transœsophagien, l'alimentation en gaz étant connectée à l'extrémité proximale du cathéter transœsophagien ; et
un système de commande à rétroaction configuré pour réguler la pression et le débit du gaz de l'alimentation en gaz à l'aiguille.

2. Système à base de cathéter pour l'administration de dioxyde de carbone au tissu fibro-graisseux entre l'œsophage et le cœur pour la prévention de dommages œsophagiens et/ou de fistules pendant une ablation intra-cardiaque de l'oreillette gauche selon la revendication 1, le dispositif de délivrance d'aiguille étant configuré pour déployer et rétracter l'aiguille dans et depuis le tissu fibro-graisseux à travers la paroi de l'œsophage.

3. Système à base de cathéter pour l'administration de dioxyde de carbone au tissu fibro-graisseux entre l'œsophage et le cœur pour la prévention de dommages œsophagiens et/ou de fistules pendant une ablation intra-cardiaque de l'oreillette gauche selon la revendication 2, le mécanisme d'ancrage fixant le cathéter transœsophagien en position dans l'œsophage.

4. Système à base de cathéter pour l'administration de dioxyde de carbone au tissu fibro-graisseux entre l'œsophage et le cœur pour la prévention de dommages œsophagiens et/ou de fistules pendant une ablation intra-cardiaque de l'oreillette gauche selon la revendication 3, le mécanisme d'ancrage comprenant un ballonnet (802) déployable à l'intérieur de l'œsophage.

5. Système à base de cathéter pour l'administration de dioxyde de carbone au tissu fibro-graisseux entre l'œsophage et le cœur pour la prévention de dommages œsophagiens et/ou de fistules pendant une ablation intra-cardiaque de l'oreillette gauche selon la revendication 4, l'aiguille étant déployable de manière proximale par rapport au ballonnet.

6. Système à base de cathéter pour l'administration de dioxyde de carbone au tissu fibro-graisseux entre l'œsophage et le cœur pour la prévention de dommages œsophagiens et/ou de fistules pendant une ablation intra-cardiaque de l'oreillette gauche selon la revendication 4, comprenant en outre un composant de sollicitation de l'aiguille (904).

7. Système à base de cathéter pour l'administration de dioxyde de carbone au tissu fibro-graisseux entre l'œsophage et le cœur pour la prévention de dommages œsophagiens et/ou de fistules pendant une ablation intra-cardiaque de l'oreillette gauche selon la revendication 6, l'aiguille étant déployable de manière distale par rapport au ballonnet par l'intermédiaire du composant de sollicitation d'aiguille.

8. Système à base de cathéter pour l'administration de dioxyde de carbone au tissu fibro-graisseux entre l'œsophage et le cœur pour la prévention de dommages œsophagiens et/ou de fistules pendant une ablation intra-cardiaque de l'oreillette gauche selon la revendication 4, comprenant en outre un moyen pour déterminer la position de l'aiguille et l'insufflation.

9. Système à base de cathéter pour l'administration de dioxyde de carbone au tissu fibro-graisseux entre l'œsophage et le cœur pour la prévention de dommages œsophagiens et/ou de fistules pendant une ablation intra-cardiaque de l'oreillette gauche selon la revendication 8, le moyen pour déterminer la position de l'aiguille et l'insufflation comprenant un capteur de position tridimensionnel (918).
